# EUROPEAN PATENT SPECIFICATION

(11) **EP 2 491 987 B1**
(45) Date of publication and mention of the grant of the patent: **13.06.2018**
(21) Application number: 10824498.9
(22) Date of filing: 21.10.2010
(51) Int. Cl.: A63B 23/18, A63B 21/02, A63B 21/04, A63B 71/06

(54) **DEVICE FOR EVALUATING AND TRAINING RESPIRATORY FUNCTION, ON BOTH INSPIRATION AND EXPIRATION**
VORRICHTUNG ZUM BEWERTEN UND TRAINIEREN DER ATEMFUNKTION BEIM EIN- UND AUSATMEN
DISPOSITIF D'ÉVALUATION ET D'ENTRAÎNEMENT RESPIRATOIRE PAR INSPIRATION ET EXPIRATION

(30) Priority: 21.10.2009 ES 200901496 U
(43) Date of publication of application: 29.08.2012
(73) Proprietor: Dolade Guardia, Josep Manel, 17256 Pals (Gerona) (ES); Fundacio Imim, 08003 Barcelona (ES)
(72) Inventor: OROZCO LEVI, Mauricio, E-08003 Barcelona (ES); GEA GIRAL, Joaquin, E-08003 Barcelona (ES)
(86) International application number: PCT/ES2010/000437
(87) International publication number: WO 2011/048244

(56) References cited:
- EP-A1- 0 372 148
- EP-A1- 1 402 915
- WO-A1-00/78407
- WO-A1-00/78407
- CN-Y- 2 317 901
- US-A- 4 226 233
- US-A- 4 226 233
- US-A- 4 533 137
- US-A- 4 739 987
- US-A- 5 566 669
- US-A- 5 649 533
- US-A- 5 649 533

## Description

### Aim of the invention

As is set out in the announcement of this descriptive report, this invention is concerned with a device for evaluating and training the respiratory muscles in humans using a dual-mechanism threshold opening valve for the imposing of both inspiratory and expiratory loads, whose objective is to measure the force and endurance of the respiratory muscles and train them if necessary. Moreover, for configuring this, its characteristics and set up represents a substantial improvement as compared to the medically apparatuses that are normally used for improving and assisting respiratory capacity, also improving the possible legal background on industrial property matters.

The objective of this invention is to develop an apparatus that makes it possible to exercise both the musculature and other elements involved in breathing, in both inhaling and exhaling, without distinction.

### State of the art

There are currently different medical apparatus used for measuring and/or improving the respiratory capacity of healthy people and people suffering from an illness. In general, the former make it possible to measure long capacities and volumes, and consist of a system for collecting the air that is breathed out by the patient in both fast and slow breathing. This air moves a signalling element that in turn indicates the respiratory capacity of that user. These types of apparatus, specifically those known as "spirometers", are extremely useful for patients with chronic bronchitis, pulmonary emphysema and bronchial asthma, because they make it possible to evaluate what the respiratory flow is at any one particular time and to check the degree of obstruction of the respiratory passageways. However, there are no apparatus that make it possible to determine the level of endurance of the respiratory muscles.

Another type of instruments are those that are used in recovering lung capacity following post-operative situations or in circumstances that require a specific form of stimulation of the lungs. In this case, the airflow produced by the patient moves visual elements that can be easily identified, such as colour spheres that are strategically placed at different levels or positions. In this way, as the lung capacity increases, the airflow produced increases and the spheres move around more, and so consequently the patient obtains mental stimulus. These instruments suffer from the defect that the endeavour involved depends on the speed that the air comes out, and therefore the user frequently reduces the utility of this unconsciously.

In spite of the wide range of medical equipment and apparatus used to evaluate pulmonary function, most of these only tend to act in the expiratory phase, that is to say, as the air is expelled from the lungs. The objective of this invention is a device with a valve that makes it possible, in the same piece of apparatus, to determine the resistance of the elements responsible for the air flow, both the inspiratory and expiratory muscles, and exercise these if this is necessary. This substantially covers its applications to different types of patients, in both the diagnosis and therapy fields.

There are some patents that develop devices that are similar to that which is proposed in this invention, with a structure that covers a casing with a central chamber with an intake nozzle for applying the patient's mouth to and some end chambers for inhaling and exhaling, incorporating certain mechanisms of resistance that the patient has to overcome. In these and other devices the internal regulation mechanism of both valves is of a resistive nature, with some limitations on its functioning. For example, in the document WO 0078407, It is described a pulmonary exercise device comprising a tubular body with chambers are arranged in an L-shaped conformation, with resilient biasing means located in the inspiratory chambers and inspiratory, having an air inlet, an air outlet and a mouthpiece, the air inlet being closed by means of a resiliently biased one way valve and the air outlet being closed by means of resiliently biased one way valve; in which the resilient bias acting against the air flow in the air inlet, or in the air outlet or in each of the air inlet and outlet is adjustable so as to enable the device to be tuned to the individual requirements of the user; in the resilient bias in the air inlet and/or the air outlet is a tension spring or a compression spring, in wich, according the tension spring or a compression spring, the adjustment of the resilient bias is provided by loading or unloading the appropriate spring so as to provide increased or decreased initial tension/compression, being each spring is removable and in which one or both of the air inlet and air outlet employ one of three springs respectively, so as to provide light, medium or heavy duty exercise, ie the valve that is presented shows three positions of resistance -minimum, medium and maximum - so as to provide, respectively, an exercise of light, medium or heavy work. These springs have to be changed whenever required vary the level of effort and therefore resistance.

The shaping of a device for breathing exercise and / or lung based on a tubular central body with an air inlet, an air outlet and a nozzle not in itself novel, being equally logic means the existence of the opening and closing of the crossings in and out of the air since it is the air flow which allows to carry out the purpose of this type of device, which is none other than the exercise capacity and pulmonary respiratory patient.

What we do must be new is precisely the technical mechanism that regulates the passage of air and establish a sufficiently broad adjustable resistance.

In the case of WO 0078407, the technical means that are claimed are three springs adjusted by a screw, so the most you get is to have three degrees or positions of strength - minimum, medium and maximum - with no possibility of a finer graduation.

Other document, US 4739987, shows a valve that has some internal membranes with a particular number of perforations or flow-through vents, which have a diameter that is increased, in such a way that the resistance that the patient has to overcome varies.

In those and other technical developments, the resistance framework to be overcome is limited, with no possibility of being gradually increased, which is precisely the aspect that is developed in the patent that is presented.

### Description of the invention

Thus, this invention is realised by means of a device for evaluating and training the respiratory muscles in humans as claimed in claim 1, which incorporates a valve with a threshold mechanism for inspiratory and expiratory endurance. Specifically, the objective of this valve is to provide fixed opening pressure (i.e. a threshold) (therefore not dependent on the air flow), both inspiratory and expiratory, with the essential feature that this pressure level can be manipulated and adjusted using an external regulator. This aspect represents a fundamental advantage as compared to valves with different threshold mechanisms, such as for example, those of a resistive type, whose degree of opening pressure cannot be assured because this depends on the patient's ventilatory pattern (i.e. respiratory frequency and current volume).

As regards the shape of this, this device is structured on the basis of an external cylindrical casing comprising three parts joined between themselves in the form of chambers, with the intermediate part or common chamber having a vent with a significant diameter, connected to an intake nozzle for the user to inhale and exhale air, while the end parts have a series of smaller vents so air can enter into the inspiratory chamber and exit the expiratory chamber. The casing is hollow inside it, with the exception of two equidistant membranes. Each one of these membranes is made up of one fixed part and a moving part, whose degree of opening and closing regulates the degree of pressure of the passage of the air. It is this fixed part of each membrane that separates the common or intermediate chamber form the corresponding end chambers. The device incorporates a regulating element or controlling part that is in joined with each one of the membranes for setting the opening pressure (i.e. the external resistance), measured in pressure units (i.e. centimetres of water, H₂O cm), and that is connected via a spring or a similar object with a moving membrane (i.e. sole-direction diaphragm) that opens or closes so as to permit air to depart and/or enter, depending on the chamber. This regulating control part is, preferably, rotational by nature, but it can work using another selection mechanism.

In this way, a simple adjustment of the rotational element works on the relevant moving membrane, regulating the degree of pressure required in order to establish the opening or closing with respect to the fixed membrane, so as to control the pressure against the air flow and the consequent resistance that it is necessary to overcome in every inhalation and in every exhalation.

This shape makes it possible for the device and corresponding valve to be used without distinction in order to generate external resistance (proportional to the air pressure of the respective diaphragm) of an inspiratory type (air entry) or expiratory type (the air leaving). The dual nature of the mechanism of the valve derives from the shape of the dual-chamber device and from the dual mechanism of membranes (independent between themselves). This means that it is possible to work with the inspiratory chamber or with the expiratory chamber, either independently or simultaneously.

As far as the membranes are concerned, as has been mentioned above, each one of these is made up of two facing parts that are fitted into a resting position, a fixed one and a moving one, which are separated by the pressure of the air expelled by the user, in accordance with the degree of turn and the consequent level of resistance established by the regulating element described.

Thus, the fixed part of the membrane, in itself, is a disc with a set of flow-through vents, four of them in principle, which make it possible for the air flow to pass and with a perimeter lowering which makes it possible to adjust this between the central or common chamber and the relevant end chamber (inspiratory or expiratory), at the same time as also performing a function as an element that separates both of these. This part faces a second moving part in the form of a disc, of a lesser thickness and that is extended along a central longitudinal appendix in which a tensioning element is fitted, in the form of a metal strip, which in turn is fitted to and interacts with the regulating rotational element. Should it be necessary, the membrane incorporates parts such as toric joints or similar items for ensuring that the unit is airtight.

The external casing, cylindrical in shape, has a lateral vent of a considerable diameter in the central chamber in which the nozzle that the patient inhales and exhales through is connected. In turn, the end chambers - inspiratory and expiratory - have a series of smaller-sized vents, placed in such a way that they connect to the outside and allow for the circulation and the passage (entry and departure) of the airflow.

The inspiratory and expiratory nozzle of the common or central chamber allows for and is extended by being coupled to a mouthpiece that makes it possible for it to be permitted into the user's mouth.

The device functions in a very straightforward way. The patient breathes in or breathes out through the central nozzle connected to the common chamber with the force necessary to overcome the level of pressure that the regulator denotes. The air flow travels through the common or intermediate chamber, it passes through the fixed membrane thanks to the existing vents, it puts pressure on the moving disc, it overcomes the force of the existing tensioning element, and it makes the aforementioned moving disc move backwards, leaving space open for the free passage of the corresponding vents through where the air communicates with the outside. In doing both inhaling and exhaling, the air flow that is breathed in or breathed out by the patient should be sufficient to establish the opening pressure necessary to overcome the resistance of the diaphragm or moving membrane. In this way, by regulating this degree of opening pressure, it forces the patient to exert greater respiratory muscular tension and to work with greater pressure and air volume.

The apparatus has greater versatility and applications as it has two airtight chambers, one for inhalation and the other for exhalation, with their respective membranes and their corresponding vents for air to enter and exit. This makes it possible for both of them to be used indistinctly or simultaneously, depending on the prescribed therapy or medical recommendations.

The features and characteristics of this valve make it possible for the device to be used in recovery therapies and in procedures for diagnosing respiratory muscular dysfunction. That is to say, by means of determining the force and resistance of both inspiratory and expiratory muscles it is possible to evaluate the degree of muscular involvement in both healthy subjects and in patients.

Amongst the therapeutic purposes, it has been proven to be preferred for people with chronic respiratory illnesses (strictly speaking, affecting the lungs, the thoracic wall or the skeletal muscles) that are associated with ventilatory complaints of diverse degrees of severity, patients with atrophied or weak musculature (cachexia or sarcopenia), which require respiratory muscular training, and healthy individuals who intend to do respiratory physical training and conditioning (i.e. elite sportspeople, sports fans). Also, for example, it is preferred in patients undergoing post-surgical rehabilitation, immediately after or subsequent to operations, those patients suffering neuro-muscular pathologies, cardiac failure, etc. in which, thanks to its capacity to regulate resistance to air flow, they can gradually increase said muscular strength and stamina, improving the patient's lung capacity and his muscular function.

As has been stated, another one of the applications of this device is as a training device for sportspeople or healthy people who have to improve and/or exercise their muscles and organs involved in the respiratory process. To do this, it is necessary to adapt the rotational device that regulates the opening pressure of the inner membrane, placing it in the indices of desired progression, which makes it possible to improve their performance with greater pressure.

The valve can be used separately and inserted into respiratory circuits, which also makes it possible to administer medicinal gases (such as oxygen, for example) at the same time as their training.

From the description it is clear that the valve described is a significant improvement on the articles that are usually found on the market because it permits the user to work individually on inhalation and expiration, with no need to remove the device in each one of the actions.

### Description of the drawings

Some sheets of paper with some drawings on them are attached, in order to assist the understanding of the innovation for which a claim is made here. These should be analysed and considered as an example and they do not imply any limitations or restrictions.
Figure 1.- View of the entirety of the device
Figure 2.- View of the fixed part of the membrane.
Figure 3.- Plan view of the fixed part of the membrane with view of the flow-through vents.

### Description of a preferred embodiment

These figures detail the configuration of the device, which has the shape of one solid unit with a mouth opening for inhaling and exhaling and a dual-chamber for air to exit through the corresponding mouth openings, which incorporates an inner mechanism that works on the basis of some membranes for regulating the resistance threshold, being this mechanism the fundamental aspect of innovation

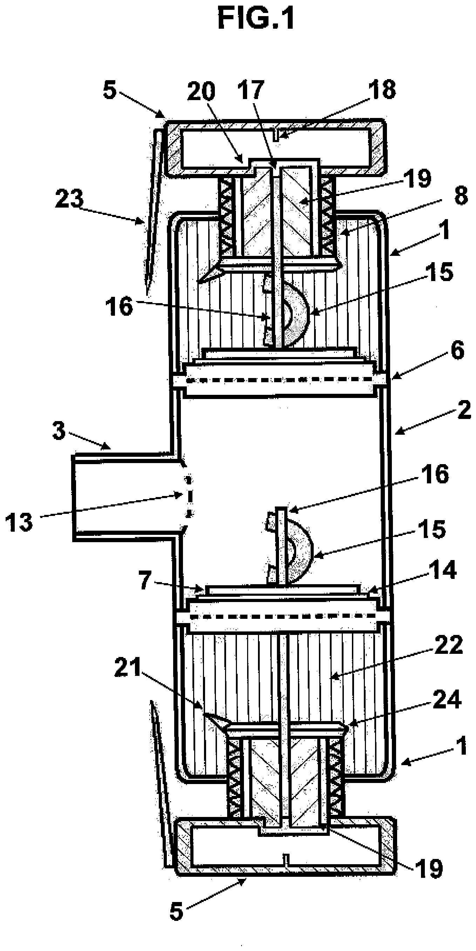

Figure 1 shows the entirety of the device for which the claim is made, comprising a cylindrical casing that covers two end chambers (1), an inspiratory one and another expiratory one and a central chamber (2) with a vent linked to a grid (13) and to an intake nozzle (3) that the user breathes in/out through. Each one of the end chambers or parts incorporates a regulating mechanism arranged in a rotational control part (5) with a diameter that fits into the perimeter framework of the device, comprising a homogenously-sized unit.

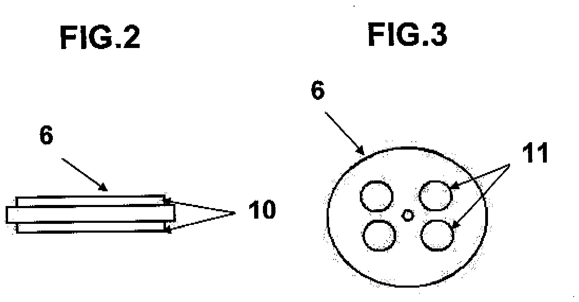

This figure 1 show the device, whose cylindrical casing is hollow inside with the exception of two equidistant membranes and the tensioning element that link them together with the respective regulating control parts. Each one of these membranes is made up of one fixed part (6) and a moving part (7), whose degree of opening or closing regulates the degree of pressure of the passage of the air by means of the regulating control part. The figure 2 an 3 show the fixed part (6) of the diaphragm or membrane, a disc with a set of flow-through vents (11), which make it possible for the air flow to pass and with a perimeter lowering (10) which makes it possible to adjust this between the common chamber (intermediate part) and each one of the end chambers (1). This fixed part of the membrane is the separator element of the common chamber, dividing the corresponding inspiratory and expiratory chambers of the device.

To summarise, it can be stated that the common or central chamber of the device is totally hollow. It is separated from the end chambers by the fixed disc of the membrane, while the end chambers (inspiratory and expiratory) are occupied by the moving part of the membrane, connected with the regulating control part via the tensioning element that is described.

In this figure 1, the same membrane as the one described is maintained, with the fixed part (6) as a separator element and the moving part (7). A silicone sheet (14) is placed between both of these. The tensioning element is arranged in a metal strip (15) attached to an axle (16) whose back end (17) is joined with a screw (18) or similar device located in the inner part of the control part (5), in such a way that when said control part is moved in a turn, the axle (16) makes the same turn, providing or removing tension from the metal strip (15). The latter in turn acts on the moving part disc (7) of the valve, adding or removing resistance to it. In the expiratory part of the device, the axle-strip is placed inside the relevant chamber, but in the inspiratory part, the axle and the strip are located inside the central chamber, and the axle is longer so as to maintain its connection -described above-with the rotational control part. In both cases the control part (5) displays a notch or protrusion (20) on its inner edge that is fitted with a lowering existing in a tubular piece (19) that surrounds the first stretch of the axle. The same control part (5) displays a second flap (21) located at the outer edge of its threaded part, whose function is to regulate the turning of the aforementioned control part as it progressively comes into contact with a series out protruding grooves (22), arranged in a perpendicular fashion inside the end chambers. A screw and bolt unit (24) is located at this back end of the threaded area of the control part (5) in the form of a cover with the function of preventing the control panel coming loose from its correct location. The same rotational control part incorporates a longitudinal external extension (23) that acts as a marker for indicating the measurement references or instructions located in the outer part of the casing.

This figure 1 shows the way that the device works. The regulating control part (5) works using the tensioning element on the moving part disc (7) or diaphragm establishing the pressure level to be overcome. When a user breathes in or breathes out using the intake nozzle (3) of the central part, the air travels through the inside of the device, passing from the central chamber to the corresponding end chamber (inspiratory or expiratory) through the flow-through vents of the fixed disc (6) of the membrane, pushing the moving disc (7), which is displaced, allowing for the air to flow from the entry or exit via the vents smallers (4) of the inspiratory or expiratory chamber. Thanks to the shape of the dual-chamber device (which determines its dual nature) and the dual-membrane mechanism (independent between themselves), it can function with just one chamber or both chambers simultaneously, depending on the test requirements.

It is not considered necessary to go into any further detail with this description in order for any expert on the matter to understand the scope of the invention and the advantages that may derive from it. The materials, shape, size and layout of the elements may be subject to variation as long as this does not entail an alteration to the essential nature of the invention. The terms on which this report has been drawn up should always be understood in a broad and non-restrictive sense, the invention being defined by the appended claims.

## Claims

1. Device for evaluating and training the respiratory muscles in humans using a dual-mechanism threshold opening valve for the imposing of both inspiratory and expiratory loads, wherein the device comprises:
- A cylindrical casing with a common central chamber (2) having a vent connected to an intake nozzle (3) for air inspiration and expiration of the user
- Two end chambers (1), one inspiratory and another expiratory, with a series of smaller vents (4) communicating the inspiratory chamber and the exhalation chamber of air with the outside
- Two inner membranes or diaphragm, equidistant from the nozzle, each having a fixed part (6) and a movable part, this fixed part of the membrane being a separator element between the common chamber and the corresponding inspiratory and expiratory chambers, each movable part being a disc (7) of lesser thickness respect to the fixed part extended into a longitudinal central appendix, being moveeable inside its respective end chamber during inhalation.
- Some regulating control parts, especially a rotational control part (5), connected to the moving part of each membrane by means of a tensioning element
**characterised in that** the tensioning elements, connected with the movable part or disc of the membrane, are two metal strips (15), each associated with a axle (16) whose terminal end engages with a screw or similar device located on the inside of each of the controls regulating parts (5), one metal strip and the first axle being located within the expiratory chamber, while the other metal strip, corresponding to the inspiratory chamber and assembly with the second axle that has a greater length than the first axle is located inside the central chamber (2) and **in that**
it comprises a silicone sheet (14) between the fixed part (6) disc and the moving disc of the membrane.

2. Device for evaluating and training the respiratory muscles in humans using a dual-mechanism threshold opening valve for the imposing of both inspiratory and expiratory loads, according to claim 1 **characterised in that** each end inspiratory and expiratory chambers (1) control part (5) displays a second flap (21) located at the outer edge of its threaded part, whose function is to regulate the turning of the aforementioned control part as it progressively comes into contact with a series out protruding grooves (22), arranged in a perpendicular fashion inside the end chambers.

3. Device for evaluating and training the respiratory muscles in humans using a dual-mechanism threshold opening valve for the imposing of both inspiratory and expiratory loads, in accordance with claim 1 and 2, **characterised in that** the rotational control part (5) fits into the perimeter frame of the device, making up a homogenously-sized unit, showing one notch or protrusion (20) on its inner edge that is fitted with an existing lowering in a tubular piece (19) that surrounds the first stretch of the axle, with a second flap (21) being located on the outer edge of its threaded part and which progressively comes into contact with the series of protruding grooves (22) arranged in a perpendicular fashion in the inner part of the end chambers, incorporating the back part of its threaded area a screw and bolt unit (24) and having a longitudinal external extension (23) that serves as a marker.

## Patentansprüche

1. Gerät zur Bewertung und zum Training der menschlichen Atemmuskulatur durch die Benutzung eines Doppelmechanismus mit Schwellenwert, der ein Öffnungsventil für die inspiratorische und exspiratorische Belastung enthält und folgendes beinhaltet:
- ein zylindrisches Gehäuse mit einer gemeinsamen zentralen Kammer (2), die eine Belüftung hat, die an eine Einströmdrüse (3) für die Ein- und Ausatmung des Benutzers angeschlossen ist
- zwei Endkammern (1), eine inspiratorische und eine exspiratorische, mit vielen kleinen Ventilen (4) zur Übermittlung an die inspiratorische Kammer, und die Ausatmungskammer der Luft mit der Außenwelt.
- zwei innere Membranen oder eine Membran, gleichweit entfernt von der Drüse, die einen beweglichen (6) und einen unbeweglichen Teil hat. Der unbewegliche Teil der Membran hat ein Trennelement zwischen der gemeinsamen Kammer und der jeweiligen inspiratorischen und exspiratorischen Kammer. Jeder bewegliche Teil ist von geringerer Scheibenstärke (7) im Verhältnis zu dem unbeweglichen Teil, der einen verlängerten und längs gerichteten zentralen Anhang hat und innerhalb der jeweiligen Endkammer während der Atmung beweglich ist.
- einige regulierende Kontrollteile, insbesondere ein Rotationskontrollteil (5), das mit dem beweglichen Teil von jeder Membran mittels eines Spannelements verbunden ist.
Das mit dem beweglichen Teil oder der Membranscheibe verbundene Spannelement zeichnet sich durch zwei Metallstreifen (15) aus, die jeweils mit einer Achse (16) verbunden sind, deren Endklammer mit einer Schraube oder einem ähnlichen Gerät an der Innenseite von jedem regulierenden Kontrollteil (5) mit einem Metallstreifen befestigt wird. Die andere Achse befindet sich innerhalb der exspiratorischen Kammer, während der andere Metallstreifen, der zu der inspiratorischen Kammer gehört und mit der zweiten Achse montiert wird, die länger als die erste Achse ist und sich innerhalb der zentralen Kammer (2) befindet. Darin befindet sich wiederum zwischen dem unbeweglichen Teil der Scheibe (6) und der beweglichen Scheibe der Membran eine Silikonmatte (14).

2. Gerät zur Bewertung und zum Training der menschlichen Atemmuskulatur durch die Benutzung eines Doppelmechanismus mit Schwellenwert, der ein Öffnungsventil für die inspiratorische und exspiratorische Belastung enthält, so wie es sich aus dem Anspruch 1 ergibt, der **dadurch gekennzeichnet ist, dass** in jedem inspiratorischen und exspiratorischen Kontrollteil (5) der Endkammer (1) eine zweite Klappe (21) angezeigt wird, die sich am äußeren Ende des Gewindeteils befindet und dessen Funktion die Regulierung der Drehung des oben genannten Kontrollteils ist, da dieses schrittweise in Kontakt mit mehreren vorstehenden Rillen (22) kommt, die senkrecht in den Endkammern angebracht sind.

3. Gerät zur Bewertung und zum Training der menschlichen Atemmuskulatur durch die Benutzung eines Doppelmechanismus mit Schwellenwert, der ein Öffnungsventil für die inspiratorische und exspiratorische Belastung enthält, so wie es sich aus dem Anspruch 1 und 2 ergibt, der **dadurch gekennzeichnet ist, dass** das Rotationskontrollteil (5) in den umlaufenden Rahmen des Geräts passt und eine Neuanfertigung einer homogenen Einheit ist. Es zeigt eine Kerbe und einen Vorsprung von Rillen (20) am inneren Ende, das mit einem bestehenden Rohr befestigt ist (19), welches das erste Stück der Achse umgibt und eine zweite Klappe (21) hat, die sich am äußeren Ende des Gewindeteils befindet und die schrittweise in Kontakt mit einigen Rillen (22) kommt, die senkrecht auf der Innenseite der Endkammern angebracht sind und die auf der Rückseite des Gewindebereichs eine Schraube, Verschlusseinheit (24) und eine längs gerichtete externe Verlängerung (23) aufweisen, die als Markierung genutzt werden kann.

## Revendications

1. Dispositif pour évaluer et entraîner les muscles respiratoires chez l'homme, en utilisant une valve d'ouverture de seuil à double mécanisme, pour imposer des charges inspiratoires et expiratoires, qui comprend :
- Un boîtier cylindrique avec une chambre centrale commune (2) qui a un évent relié à une buse d'admission (3) pour l'inspiration de l'air et l'expiration de l'utilisateur.
- Deux chambres d'extrémité (1), l'une inspiratoire et l'autre expiratoire, avec une série de petits évents (4) qui communiquent avec la chambre inspiratoire et la chambre d'expiration de l'air vers l'extérieur.
- Deux membranes internes ou diaphragmes, équidistantes de la buse, comportant chacune une partie fixe (6) et une partie mobile, cette partie fixe de la membrane étant un élément séparateur entre la chambre commune et les chambres inspiratoires et expiratoires correspondantes, chaque partie mobile étant un disque (7) d'une épaisseur inférieure par rapport à la partie fixe, prolongée en appendice central longitudinal, en pouvant être déplacée à l'intérieur de sa chambre d'extrémité respective pendant l'inhalation,
- Des parties de contrôle de la régulation, notamment une pièce de contrôle rotationnel (5) connectée à la partie mobile de chaque membrane au moyen d'un élément de tension
**caractérisé en ce que** les éléments de tension, connectés avec la partie mobile ou disque de la membrane, sont deux bandes métalliques (15), chacune associée à un axe (16) dont l'extrémité du terminal s'engage avec une vis ou un dispositif similaire, situé à l'intérieur de chacune des pièces de contrôle de la régulation (5), une bande de métal, le premier axe étant placé à l'intérieur de la chambre expiratoire, tandis que l'autre bande de métal, correspondant à la chambre inspiratoire et à l'assemblage avec le deuxième axe, qui a une longueur supérieure à celle du premier axe, est situé à l'intérieur de la chambre centrale (2) et comprend une feuille de silicone (14) entre le disque de la partie fixe (6) et le disque mobile de la membrane.

2. Dispositif pour évaluer et entraîner les muscles respiratoires chez l'homme, en utilisant une valve d'ouverture de seuil à double mécanisme, pour imposer des charges inspiratoires et expiratoires, selon la revendication 1, il se **caractérise en ce que** chaque pièce de contrôle (5) de la chambre (1) finale inspiratoire et expiratoire présente une seconde valve (21) situé sur le bord extérieur de sa partie filetée, dont la fonction est de réguler la rotation de la pièce de contrôle précitée, au fur et à mesure qu'elle entre progressivement en contact avec une série de rainures saillantes (22) disposées perpendiculairement à l'intérieur des chambres finales.

3. Dispositif pour évaluer et entraîner les muscles respiratoires chez l'homme, en utilisant une valve d'ouverture de seuil à double mécanisme, pour imposer des charges inspiratoires et expiratoires, selon la revendication 1 et 2, il se **caractérise en ce que** la pièce de contrôle rotationnel (5) s'insère dans le cadre périphérique de l'appareil, en constituant ainsi une unité d'une taille homogène, qui présente une encoche ou saillie (20) sur son bord intérieur muni d'un rabaissement présent dans une pièce tubulaire (19) entourant la première partie de l'axe, avec un deuxième volet (21) situé sur le bord extérieur de son filetage et qui vient progressivement au contact avec la série de rainures en saillie (22) disposées de manière perpendiculaire, dans la partie intérieure des chambres d'extrémité, en incorporant la partie arrière de sa zone filetée avec une unité à vis et à boulon (24) et qui a une extension externe longitudinale (23) qui sert de marqueur.
